# EUROPEAN PATENT APPLICATION

(11) **EP 2 932 974 A1**
(43) Date of publication of application: **21.10.2015**
(21) Application number: 14164688.5
(22) Date of filing: 15.04.2014
(51) Int. Cl.: A61K 33/06, A61K 33/42, A61P 19/08, A61P 19/10

(54) **Composition for prophylaxis and treatment of bone disorders**

(71) Applicant: Chemische Fabrik Budenheim KG, 55257 Budenheim (DE)
(72) Inventor: Müller, Werner E. G., DE - 65203 Wiesbaden (DE); Wang, Xiaohong, DE - 55129 Mainz (DE); Schröder, Heinz C., DE - 65189 Wiesbaden (DE)
(74) Representative: WSL Patentanwälte Partnerschaft mbB

(57) **Abstract**

A composition for use in medicine or as a dietary supplement, the composition comprising at least one complex or salt of trivalent metal cation (Me³⁺) with inorganic polyphosphate (polyP), wherein the trivalent metal cation (Me³⁺) is selected from the elements of the group consisting of Al, Ga, In, La, Ce, Pr, Nd, Sm, Eu, Gd, Tb, Dy, Ho, Er, Tm, Yb and Lu.

## Description

### Field of the invention

The present invention relates to a composition that is particularly useful in medicine or as a dietary supplement for use in the prophylactic or therapeutic treatment of osteoporosis or bone disorder.

### Background of the invention

Bone formation and maintenance are controlled in a balanced interplay of osteoblasts (bone-forming cells) and osteoclasts (bone-degrading cells). A disorder of this balance results in osteoporosis (hyperactivity of osteoclasts) or osteopetrosis (hyperfunction of osteoblasts). The tuned interaction between these cell types is under the control of cell adhesion molecules (integrins), and soluble intra- and extracellular organic factors and their corresponding receptors. In addition, inorganic mineralic deposits, e.g. hydroxyapatite (HA) or calcium carbonate, induce substances of organic nature and, by that, modulate the differentiation of the bone precursor cells to functionally active osteoblasts and osteoclasts. Besides these organic mediators, inorganic polymers, e.g. biosilica/silicate and polyphosphates, influence bone metabolism.

Inorganic polyphosphates are synthesized in biological systems from ATP in enzymatic reactions by some microorganisms and metazoans. Depending on the counter-ion the biologically synthesized inorganic polyphosphates occur in biological systems either in the soluble, amorphous or crystalline state. It has previously been reported that inorganic polyphosphate modulates hydroxyapatite synthesis in the *in vitro* SaOS-2 cell system. (Leyhausen G, Lorenz B, Zhu H, Geurtsen W, Bohnensack R, Müller WEG, Schröder HC (1998) Inorganic polyphosphate in human osteoblast-like cells. J Bone Mineral Res 13:803-812). After exposure of SaOS-2 with inorganic polyphosphates of different chain lengths, and complexed with Ca²⁺, the expression of the bone-cell specific alkaline phosphatase (ALP), an enzyme that had been implicated in phosphate metabolism in bone, is induced (Müller WEG, Wang XH, Diehl-Seifert B, Kropf K, Schloßmacher U, Lieberwirth I, Glasser G, Wiens M and Schröder HC (2011) Inorganic polymeric phosphate/polyphosphate as an inducer of alkaline phosphatase and a modulator of intracellular Ca2+ level in osteoblasts (SaOS-2 cells) in vitro. Acta Biomaterialia 7:2661-2671). In SaOS-2 cells ALP becomes upregulated allowing a facilitated hydrolysis of polyphosphate at the spot, where inorganic phosphate (Pi) is used as a substrate for hydroxyapatite formation. The assumption of a potential osteogenic influence of inorganic polyphosphate is based on the finding that in SaOS-2 cells, incubated with inorganic polyphosphate, not only an increased expression of bone morphogenetic protein-2 (BMP2) occurs but also an inhibition of phosphorylation of factor IκBα that is supposed to abolish RANKL-mediated NF-κB activation in RAW 264.7 cells (Wang XH, Schröder HC, Diehl-Seifert B, Kropf K, Schlosmacher U, Wiens M, Müller WEG (2012) Dual effect of inorganic polymeric phosphate/polyphosphate on osteoblasts and osteoclasts in vitro. J Tissue Engineer Regen Med; doi: 10.1002/term.1465). Besides of being a source for the supply of inorganic phosphate (Pi), required for the hydroxyapatite synthesis by osteoblasts, inorganic polyphosphate is suspected to function as scaffold for bone tissue engineering (Baksh D, Davies JE, Kim S (1998) Three-dimensional matrices of calcium polyphosphates support bone growth in vitro and in vivo. J Mater Sci Mater Med 9:743-748).

### Object of the invention

It was an object of the present invention to provide a substance or a composition showing improved properties in the prophylactic or therapeutic treatment of osteoporosis or bone disorder. It was a further object of the present invention to provide an improved method for the prophylactic or therapeutic treatment of osteoporosis or bone disorder.

### Description of the invention

The present invention provides a composition for use in medicine or as a dietary supplement, the composition comprising at least one complex or salt of trivalent metal cation (Me³⁺) with inorganic polyphosphate (polyP), wherein the trivalent metal cation (Me³⁺) is selected from the elements of the group consisting of Al, Ga, In, La, Ce, Pr, Nd, Sm, Eu, Gd, Tb, Dy, Ho, Er, Tm, Yb and Lu.

The inventors of the present invention have surprisingly found that inorganic polyphosphates (polyP) in a complex or salt with specifically selected trivalent cations (Me³⁺), designated herein also as polyP•Me, causes a superior biological effect on bone formation as compared to the single components polyP and Me³⁺ ions. PolyP•Me causes an induction of hydroxyapatite formation in bone-forming cells and an induction of the expression of the genes encoding BMP2 and collagen type I. The morphogenetic potential of polyP•Me can be applied in treatment of bone disorders, such as bone fractures, bone defects and osteoporosis, and for bone tissue engineering approaches.

The experiments carried out by the present inventors have shown that exposure of SaOS-2 cells to polyP•Me according to the present invention causes a distinct increase in hydroxyapatite formation. It could be shown that alkaline phosphatase (ALP), expressed by SaOS-2 cells, becomes activated if the cells are incubated with polyP or polyP•Me. ALP degrades polyP•Me at the location around those osteoblast-like cells. The oligo- or mono-phosphate units resulting from the degradation of the polyphosphate by ALP, as well as the Me³⁺ ions, separated from the polyphosphate due to its degradation, display biological function in the vicinity of the SaOS-2 cells separately from each other. While polyP and it hydrolysis products serve as substrates for hydroxyapatite formation, the Me³⁺ ions cause an initiation of a differentiation pathway for osteoblasts with a sequential expression of BMP2, ALP, and collagen type I.

According to the present invention, polyP•Me acting as a morphogenetically active polymer complex can be used in prophylactic or therapeutic treatment of osteoporosis or bone disorder in humans and animals, and for bone and tissue synthesis and biomedical engineering.

In the experiments the inventors have compared the biological activity of polyP in a complex or salt with Me³⁺ (herein designated as polyP•Me) with the biological activity caused by polyP (in the form of its calcium salt) and by Me³⁺ (in the form of its Me(III) salt, MeCl₃) alone regarding their potencies to induce hydroxyapatite (HA) formation in SaOS-2 cells *in vitro.* The three compounds, Me(III) salt (e.g. MeCl₃), polyP and polyP•Me are non-toxic at concentrations up to at least 30 µM. The inventors unexpectedly found that at a low concentration of 5 µM polyP•Me significantly induced hydroxyapatite (HA) formation arranged in a nest-like pattern, as determined by Alizarin Red S staining and by quantitative determinations using that dye. PolyP and Me(III) salt (e.g. MeCl₃) at 5 µM each also induced hydroxyapatite (HA) formation, however, to a lesser extent. Energy-dispersive X-ray spectroscopy (EDX) and EDX line scanning revealed that, besides of P and Ca and other biogenic elements, the hydroxyapatite (HA) crystals did not contain any traces of the Me atoms. The inventors found that exposure of cells to polyP•Me resulted in a strong increase in alkaline phosphatase (ALP) activity; whereby this enzyme did not cause a distinct degradation of polyP but of polyP•Me which was extensively hydrolyzed. The morphogenetic activity of the introduced trivalent Me³⁺ cations, in the complexed form of polyP•Me, is underscored by the finding of a strong upregulation of the genes encoding BMP2 as well as collagen type I.

Accordingly, the composition of the present invention is suitable for use in the prophylactic or therapeutic treatment of osteoporosis or bone disorder. The composition can be administered to a human or animal in any suitable form as a pharmaceutical or medical product or as a dietary supplement.

The medical indication "osteoporosis or bone disorder", as used in the present application, shall comprise all forms of osteoporosis and bone disorders currently known in the medical field. For example, osteoporosis includes age-related primary osteoporosis, idiopathic juvenile osteoporosis and osteosclerosis, all types of secondary osteoporosis that may have a large scope of causes. Secondary osteoporosis may be caused by the therapeutic application of steroids, immunological events (systemic lupus erythematodes; chronic polyarthritis), oncological events (plasmacytoma, mastocytosis, chronic lymphocytic leukemia), metabolic reasons (cystic fibrosis, diabetes mellitus) or endocrine disorders (morbus cushing, acromegaly, hypogonadism). The indication does further include osteogenesis imperfecta (sometimes known as brittle bone disease, or Lobstein syndrome), Paget disease of bone (morbus Paget) or hypophosphatasia (Rathbun syndrome).

The application of the present invention may also include the repair and treatment of bone defects, the preparation of bone implants or bone substitutes, for example for use in bone elongation or cosmetic or reconstructive surgery. The present invention may also be applied in biomedical bone tissue engineering and for the prophylactic or therapeutic treatment of the mineral metabolism, such as hyper or hypo calcification of bone tissue or non-bone organs, such as blood vessels. The present invention is generally applicable in any prophylactic or therapeutic treatment or any system promoting bone synthesis and/or inhibiting bone degradation in vivo or in vitro.

In a preferred embodiment of the present invention, the composition further comprises pharmaceutically suitable carriers and/or additives selected from poly(lactic acid) (PLA) microspheres, poly(lactide-co-glycolide) (PLGA) microspheres, poly(lactide-co-glycolide-glucose) (PLG-GLU) microspheres, gelatin, calcium phosphate cements, poly(lactide-co-glycolide) (PLGA) nanoparticles, polyethylene oxide (PEO) microspheres, carbomer microspheres, chitosan microspheres, poly(lactide-co-caprolactone) microspheres, calcium phosphate microspheres, liposomes, alginate beads, chitosan beads, or combinations of the afore-mentioned. However, the afore-mentioned suitable carriers and additives are not intended to limit the scope of the present invention, and other suitable carriers and/or additives that are known in the art can be used in combination with the composition of the present invention, depending on the administration route of the composition.

For implantation of the composition of the present invention, suitable carriers and/or additives are, for example, poly(lactic acid) (PLA) microspheres, poly(lactide-co-glycolide) (PLGA) microspheres, or blends of PLA with PLGA, poly(lactide-co-glycolide-glucose) (PLG-GLU) microspheres, gelatin and calcium phosphate cements. For oral administration of the composition of the present invention, suitable carriers and/or additives are, for example, poly(lactide-co-glycolide) (PLGA) nanoparticles, polyethylene oxide (PEO) microspheres, carbomer microspheres and chitosan microspheres.

Also, Eudragit® (trademark of Evonik Industries), a series of products of Evonik Industries comprising inorganic copolymers based on methacrylic acid and acrylate, such as methyl methacrylate or ethyl acrylate, are suitable carriers and/or additives for the oral administration of the composition of the present invention. The suitable Eudragit® products include anionic copolymers based on methacrylic acic and methyl methacrylate, cationic copolymers based on dimethylaminoethyl methacrylate, butyl methacrylate and methyl methacrylate, and copolymers of ethyl acrylate, methyl methacrylate and methacrylic acid ester with quaternary ammonium groups, or mixtures of other methacrylate derivatives.

Suitable carriers and/or additives for the local delivery of the composition of the present invention are, for example, poly(lactide-co-caprolactone) microspheres or calcium phosphate microspheres, and for local injection, poly(lactic acid) (PLA) microspheres, poly(lactide-co-glycolide) (PLGA) microspheres, liposomes, alginate beads or chitosan beads are suitable carriers and/or additives.

The composition of the present invention can preferably be formulated for oral, parenteral or topic administration or for administration by subcutaneous injection, intravenous injection, intraarterial injection, intraossal injection, intravertebral injection, intraarticular injection, intramuscular injection. The composition of the present invention can also be administered in any other suitable form, depending on the indication and application, for example as a bio compatible coating to bones, teeth or tissue. The composition of the present invention can also be applied as an active ingredient in implant material, such as bone cements used, for example, to fill free spaces in the bone caused by accident or any disease, or to fill free spaces between the bone and a prosthesis for anchoring the same.

According to an embodiment of the present invention the inorganic polyphosphate (polyP) molecules of the inventive composition consist of an average number of from 2 to 1000 phosphate units, preferably from 4 to 100 phosphate units, more preferably from 10 to 70 phosphate units. PolyP with an average number of 10 to 70 phosphate units is most effective, followed by polyP with an average number of 4 to 100 phosphate units and polyP with an average number of 2 to 1000 phosphate units. The solubility of complexes or salts of polyP with trivalent cations decreases with increasing chain lengths. Therefore the lower efficiency of polyP with an average number of more than 70 phosphate units or more than 100 phosphate units may be partially caused by the lower solubility of these complexes or salts.

According to another embodiment of the present invention the inorganic polyphosphate (polyP) molecules consist of linear polyphosphate chains, even though branched polyphosphates may also be used. However, branched polyphosphates are less stable and are more rapidly hydrolyzed, in contrast to linear polyphosphates which are hydrolysed extremely slowly in aqueous solution at neutral pH and room temperature.

According to still another embodiment of the present invention the stoichiometric ratio of the inorganic polyphosphate (polyP) and the trivalent metal cations (Me³⁺) in the complex or salt is from 5:1 to 1:1, preferably from 4:1 to 2:1, most preferably about 3:1. If the stoichiometric ratio of the inorganic polyphosphate (polyP) and the trivalent metal cations (Me³⁺) is too high, the effect on mineralization decreases. This might be partially caused by sequestration of calcium ions, which are required for HA formation by complex formation of polyP. If the stoichiometric ratio of the inorganic polyphosphate (polyP) and the trivalent metal cations (Me³⁺) is too low, the effect on mineralization also decreases. This might be partially caused by the formation of precipitates, which can be observed at concentrations of polyP and Me³⁺ higher than equimolar depending on the type of trivalent metal cation and the concentrations of polyP and Me³⁺.

It is particularly preferred if the trivalent metal cations (Me³⁺) are selected from the elements of the group consisting of Al, La, and Gd, whereby Gd has shown to be most effective and is thus most preferred.

The inventors have found that the effect of the composition of the present invention can be further increased if the composition additionally comprises calcium ions. Thus, in another embodiment of the present invention the composition further comprises calcium ions (Ca²⁺). The preferred concentrations of calcium ions are in the range of 0.3 - 30 µM, but even lower and higher concentrations of this divalent cation may be effective.

The inventors have further found that the effect of the composition of the present invention can be increased if the composition additionally comprises silicic acid in the form of monomeric silicic acid, polymeric silicic acid or combinations thereof. Thus, in another embodiment of the present invention the composition further comprises silicic acid in the form of monomeric silicic acid, polymeric silicic acid or combinations thereof. The preferred concentrations of silicic acid in the form of monomeric silicic acid, polymeric silicic acid or combinations thereof are in the range of 3 to 100 µM (monomeric silicic acid) and 100 - 400 µM (polymeric silicic acid; based on silicic acid units), but even lower and higher concentrations of silicic acid in the form of monomeric silicic acid, polymeric silicic acid or combinations thereof may be effective.

If the composition of the present invention comprises further constituents in addition to the at least one complex or salt of trivalent metal cation (Me³⁺) with inorganic polyphosphate (polyP), tha latter should be contained in the composition of the present invention in an amount of from 0.5 to 40 % by weight, preferably in an amount of from 1 to 30 % by weight, more preferably in an amount of from 2 to 20 % by weight, still more preferably in an amount of from 5 to 15 % by weight. If the amount of the at least one complex or salt of trivalent metal cation (Me³⁺) with inorganic polyphosphate (polyP) in the composition is too low, the desired effect may not be achieved.

The present invention encompasses also the use of the inventive composition, as described herein, for the prophylactic or therapeutic treatment of osteoporosis or bone disorder, as well as a method of the prophylactic or therapeutic treatment of osteoporosis or bone disorder including the administration of the inventive composition to a human or animal.

The invention will now be described further by the following examples and the accompanying figures, however, the invention is not construed to be limited thereto.

### Description of the figures

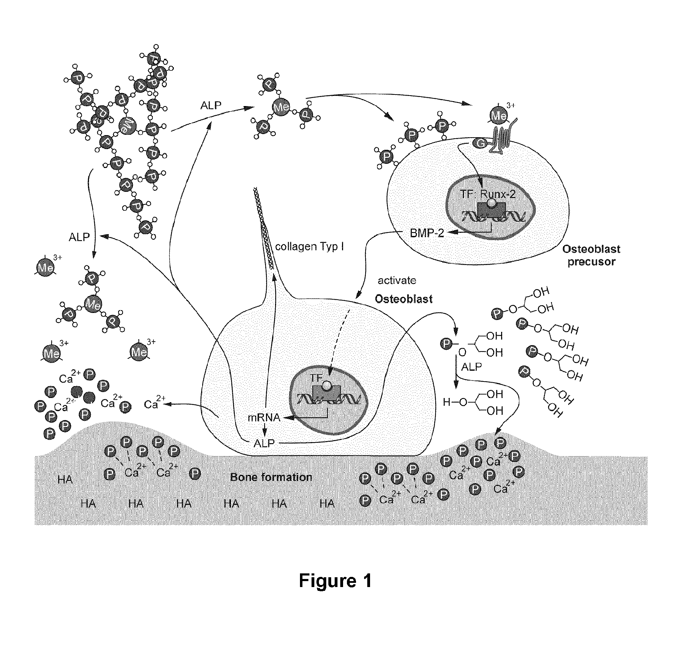

**Figure 1** shows a schematic representation of the inventor's understanding of the effect of polyP•Me on development and function of osteoblasts. It is assumed that polyP•Me is degraded by ALP (alkaline phosphatase) into oligo- or mono-phosphate units. After dissociation of Me³⁺ from the oligoP and mono-phosphate the Me³⁺ cation stimulates the extracellular calcium-sensing receptor (ECSR) that activates in an intracellular signal transduction pathway, ultimately resulting in an increased expression of *BMP2.* This mediator is then released into the extracellular space and contributes to the development of osteoblast precursor cells to mature functional osteoblasts. In those cells the expression of the genes encoding for ALP and collagen type I is induced. While the ALP is involved in the degradation of polyP•Me and also of β-glycerophosphate (ß-GP), collagen type I changes the morphology of the cells and their fibrils become extruded into the extracellular space. Finally, deposition of HA (hydroxyapatite) occurs under consumption of phosphate and Ca²⁺, resulting in an increased bone formation.

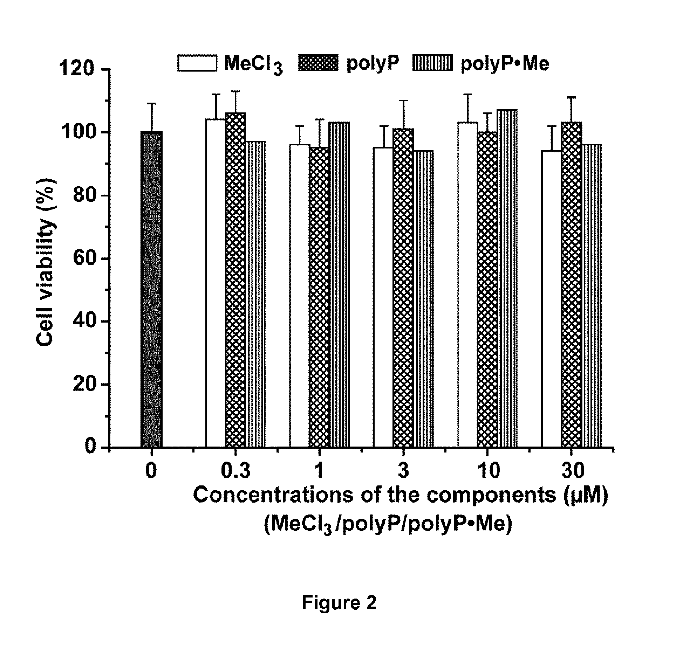

**Figure 2** shows an MTT (3-(4,5-dimethylthiazol-2-yl)-2,5-diphenyltetrazolium bromide) colorimetric assay applied to assess the viability and proliferation of SaOS-2 cells *in vitro* upon the addition of MeCl₃ alone (open bars), polyP alone (cross-hatched bars) or polyP•Me (striped bars) at different concentrations in the range from 0.3 to 30 µM. In this assay Me was Gd. The recordings of developed formazan dye in the control (filled bar) were set to 100%. The results are expressed as means (n=10 experiments each) ± SEM. None of the additions significantly affected growth and viability of the cells.

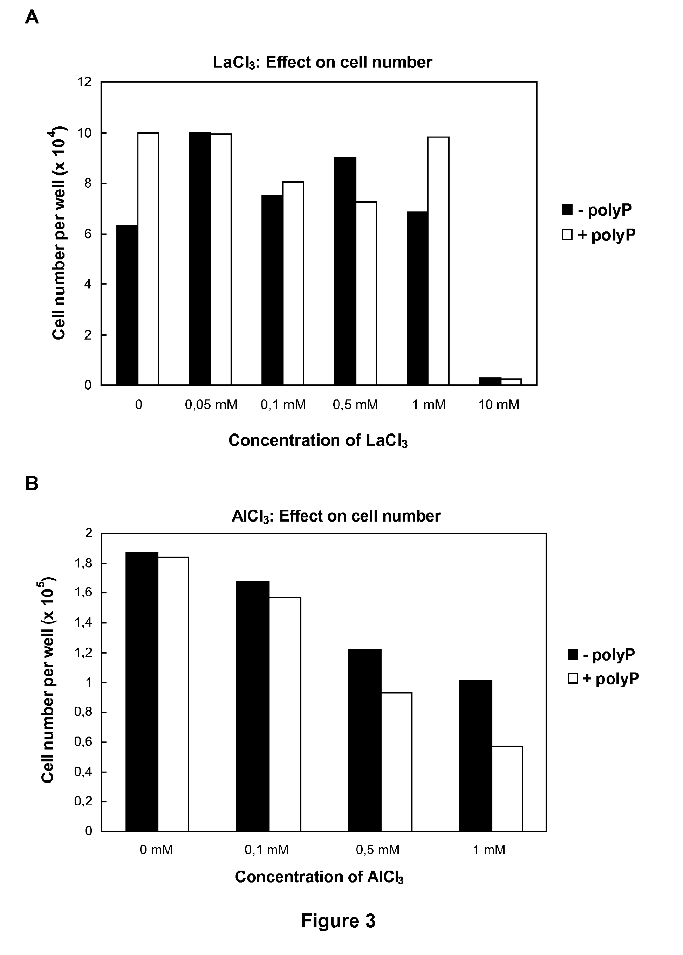

**Figure 3** shows the effects of increasing concentrations of LaCl₃ (Figure 3 A) or AlCl₃ (Figure 3 B) in the absence (filled bars) or presence (open bars) of polyP at a concentration of 50 µM on the number of SaOS-2 cells per well. Addition of LaCl₃ alone (filled bars) and addition of LaCl₃ plus polyP (open bars) did not significantly affect the cell number at concentrations from 0.05 mM to 1 mM LaCl₃, while addition of AlCl₃ alone (filled bars) and addition of AlCl₃ plus polyP (open bars) resulted in a strong reduction of cell number already at concentrations of 0.1 mM AlCl₃ and higher.

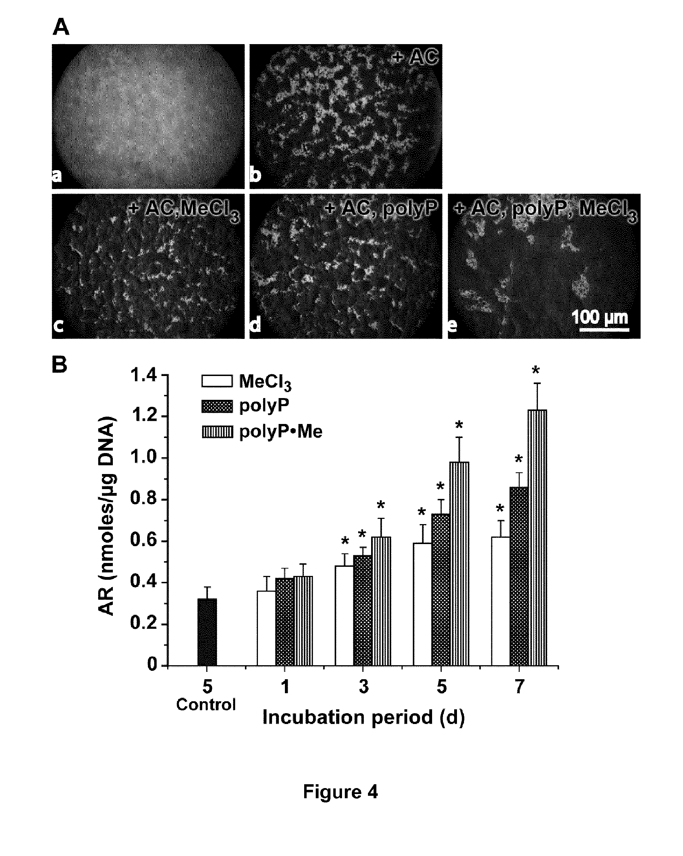

**Figure 4** shows the influence of MeCl₃, polyP, and polyP•Me on HA mineralization by SaOS-2 cells. In this assay Me was Gd. The cells were incubated with 5 µM of each of the compounds. Figure 4 A: The cells were grown for 7 d in the (a) absence or (b) presence of activation cocktail (AC = 50 µM ascorbic acid, 10 nM dexamethasone and 1 mM β-glycerophosphate). In separate experiments the cultures were incubated with the activation cocktail and additionally with (c) MeCl₃, (d) polyP, or (e) polyP•Me. After incubation, the cell assays were stained with Alizarin Red S to assess the degree of magnification. The diameter of the wells was 15 mm. Figure 4 B: To quantitatively determine the HA mineralization in SaOS-2 cells, the cultures were assayed at the end of the incubation with Alizarin Red S (AR) in the spectrophotometric assay. The results are documented; standard errors of the means are shown (n = 5 experiments per time point). *P < 0.05.
   The intensity of the Alizarin Red colorimetric reaction at the used concentration range is proportional to the extent of mineralization (HA formation), as determined by using a calibration curve. The values measured (given in nmoles of Alizarin Red S bound) were normalized to the total amount of DNA (given in µg) which is proportional to cell number (the amount of DNA per cell is assumed to remain constant during the experiment). Thereby, possible effects of the complexes or salts of polyP with trivalent cations on mineralization (HA formation) caused by changes in cell number are eliminated. The cellular DNA has been measured in parallel cultures, using the PicoGreen assay (Wiens M, Wang XH, Schloßmacher U, Lieberwirth I, Glasser G, Ushijima H, Schröder HC, Müller WEG (2010) Osteogenic potential of bio-silica on human osteoblast-like (SaOS-2) cells. Calcif Tissue Int 87:513-524).

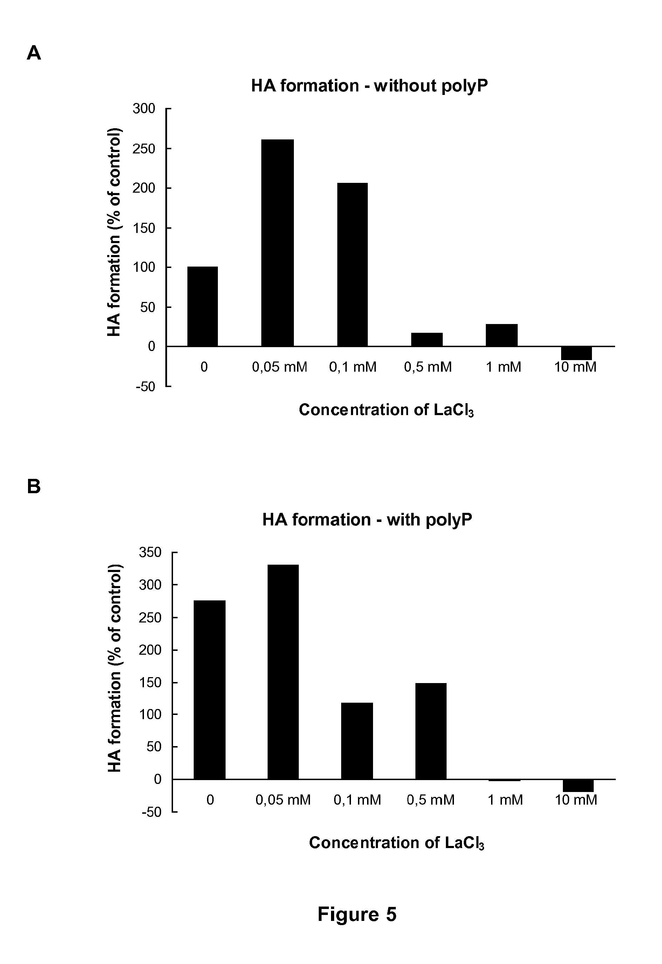

**Figure 5** shows the influence of LaCl₃, polyP, and polyP•La on HA mineralization by SaOS-2 cells. The cells were grown for 4 days in the absence of the activation cocktail (AC) and then for further 7 days (i) in the absence or (ii) in the presence of the activation cocktail with increasing concentrations of LaCl₃ without (A) or with (B) 50 µM polyP (polyP•La complex). At the end of the incubation period, HA mineralization was determined by incubation of the cultures with Alizarin Red S in the spectrophotometric assay. Figure 5 A shows HA formation in the presence of increasing concentrations of LaCl₃ without addition of 50 µM polyP. Figure 5 B shows HA formation in the presence of increasing concentrations of LaCl₃ with addition of 50 µM polyP (polyP•La complex). The OD values at 570 nm measured in the spectrophotometric assay in the absence of the activation cocktail had been subtracted from the OD values at 570 nm measured in the presence of the activation cocktail, and the HA formation in the absence of LaCl₃ and polyP (Figure 5 A) was set to 100%.

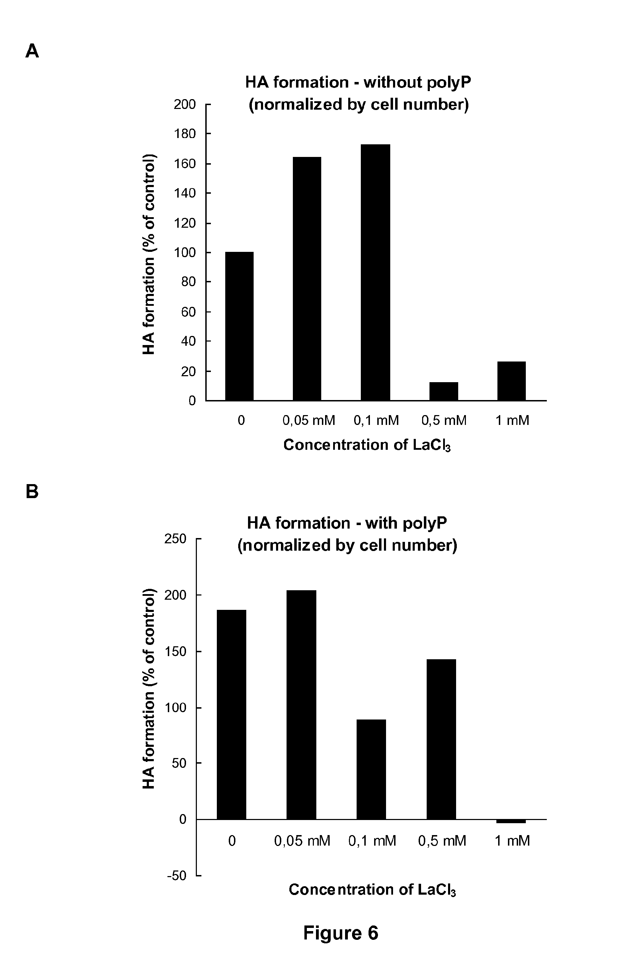

**Figure 6** shows the influence of LaCl₃, polyP, and polyP•La on HA mineralization by SaOS-2 cells, normalized by cell number. The data shown in Figure 5 had been normalized by the number of cells which had been determined in parallel assays. Figure 6 A shows HA formation in the presence of increasing concentrations of LaCl₃ without addition of 50 µM polyP, normalized by cell number. Figure 6 B shows HA formation in the presence of increasing concentrations of LaCl₃ with addition of 50 µM polyP (polyP•La complex), normalized by cell number. The HA formation in the absence of LaCl₃ and polyP (Figure 6 A) was set to 100%.

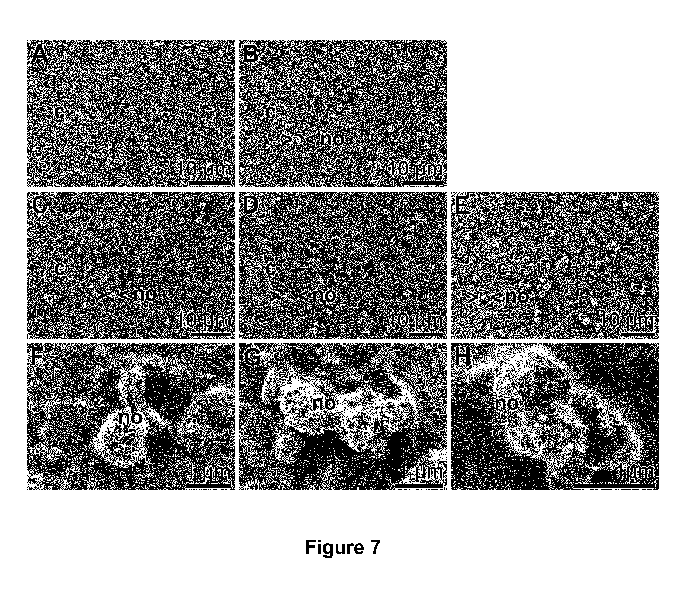

**Figure 7** shows the effect of MeCl₃, polyP, and polyP•Me on the formation of HA nodules by SaOS-2 cells. In this assay Me was Gd. 5 µM of each compound were added to the cells, and the cells were analysed by SEM. The cell cultures were incubated for 5 days either **(A)** in the absence or **(B)** in the presence of the activation cocktail. **(C)** and **(F)** show incubation of the activated cells with MeCl₃; **(D)** and **(G)** show incubation of the activated cells with polyP, and **(E)** and **(H)** show incubation of the activated cells with polyP•Me. Some nodules (no) and cell layers (c) are marked.

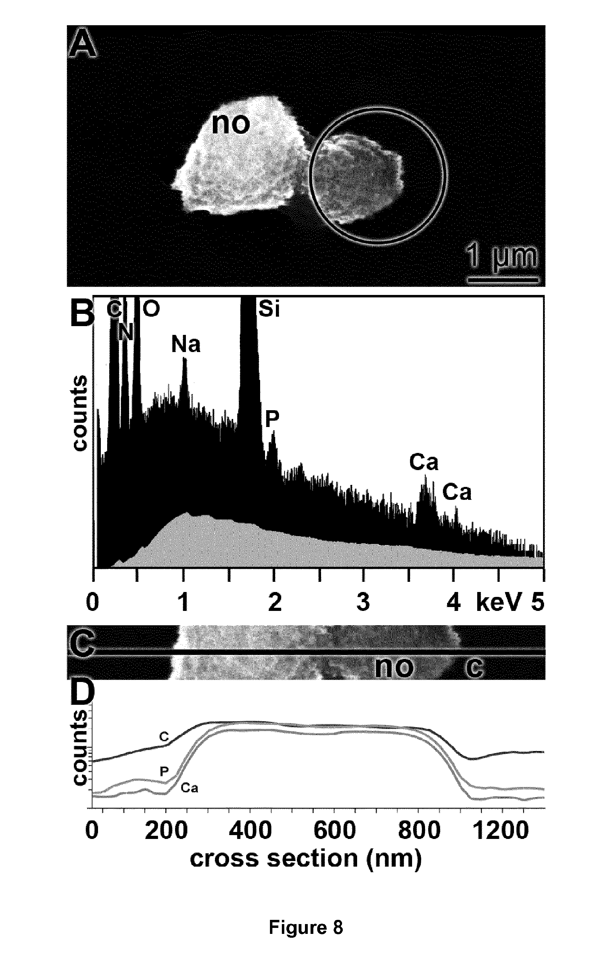

**Figure 8** shows the element distribution in a layer of SaOS-2 cells that had been incubated with polyP•Me. In this assay Me was Gd. Cells were grown for 7 days in the activation cocktail together with 5 µM polyP•Me. Figure 8 A shows an SEM, wherein the circle marks the area with a nodule (no) which had been analyzed by EDX. Figure 8 B shows the EDX spectrum of the area marked in Figure 8 A. The EDX spectrum highlights the biogenic elements and also P and Ca. Gray areas represent the spectra originating from the Bremsstrahlen. Figure 8 C shows a magnification of an area along a nodule (no) by SEM imaging. Figure 8 D shows an EDX line-scan profile performed along the indicated line in Figure 8 C, showing the relative intensities of C, P and Ca as a function of position. The nodule area (no) and the cellular surroundings (c) are marked.

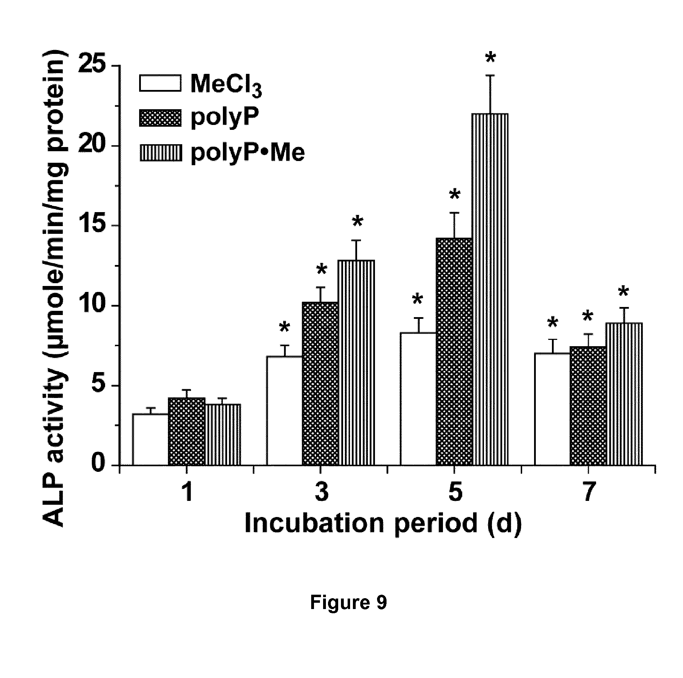

**Figure 9** shows the increase in ALP enzyme activity in SaOS-2 cells after treatment with MeCl₃ (open bars), polyP (cross-hatched bars) and polyP•Me (striped bars). In this assay Me was Gd. The activated cells were incubated with 5 µM of one of the compounds separately, or together. After an incubation period of 1 to 7 days the cells were broken and the extracts were measured for ALP activity. Standard errors of the means are shown (n = 6 experiments per time point). *P < 0.05.

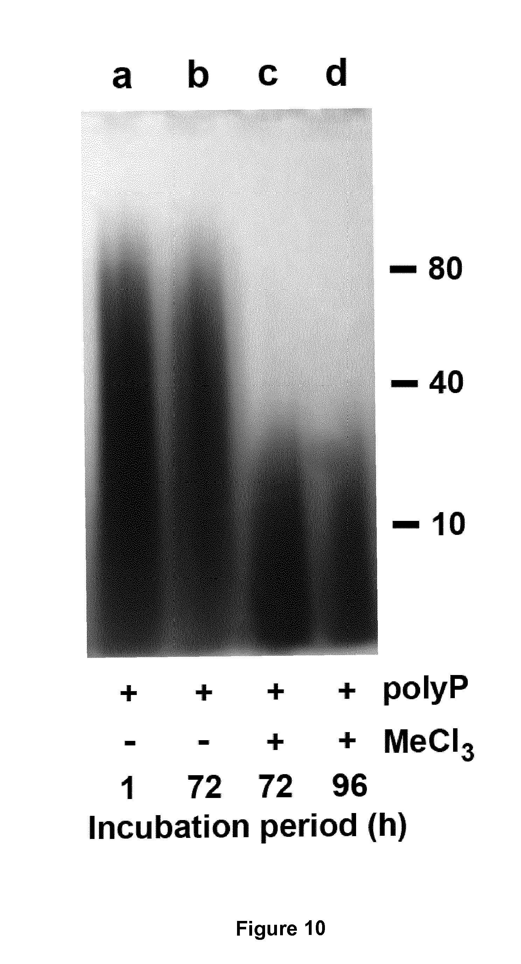

**Figure 10** shows the degradation of polyP after incubation with medium from SaOS-2 cells that had been incubated for 5 days with 5 µM polyP•Me. Either polyP directly or complexed polyP•Me had been incubated with the medium for 1 h or 72 or 96 h. In this assay Me was Gd. Then the samples were analyzed by urea polyacrylamide gel electrophoresis, as described under material and methods. Migrations of the polyP standards with a chain length of 80, 40, or 10 phosphate units were used as markers.

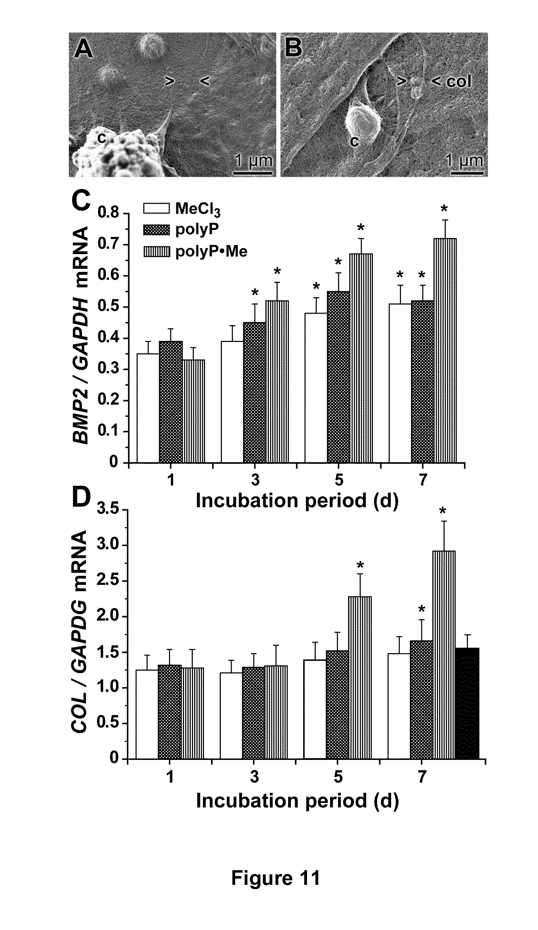

**Figure 11** shows in Figures 11 A and B SEM pictures of SaOS-2 cells after incubation in medium that had been supplemented with activation cocktail and **(A)** 5 µM polyP, or **(B)** 5 µM polyP•Me for 10 days. In figure 11 B it can be observed that the polyP•Me treated cell shows a pronounced spindle-shape and many collagen fibrils (><col), while those morphological characteristics are missing in the polyP-treated cell (><) shown in figure 11 A. Figures 11 C and D show the effect of polyP, polyP•Me or MeCl₃ on *BMP2* **(C)** and *collagen type I* **(D)** gene expression.
SaOS-2 cells were incubated with these compounds for 1, 3, 5, or 7 days. Then RNA was extracted from the cultures and the steady-state levels of the respective transcripts were quantified by qRT-PCR. In parallel the transcript level of *GAPDH* was determined and used as reference for normalization. The expression level of the genes after exposure to Me³⁺ (open bars), polyP (cross-hatched bars) and polyP•Me (striped bars) are given as a function to the one of *GAPDH.*
In addition, the expression level of *collagen type I* as a function to the one of *GAPDH* after incubation of the cells in the absence of Me³⁺, polyP and polyP•Me is shown in **D** (filled bar). n = five experiments per time point; *P < 0.05. In these assays Me was Gd.

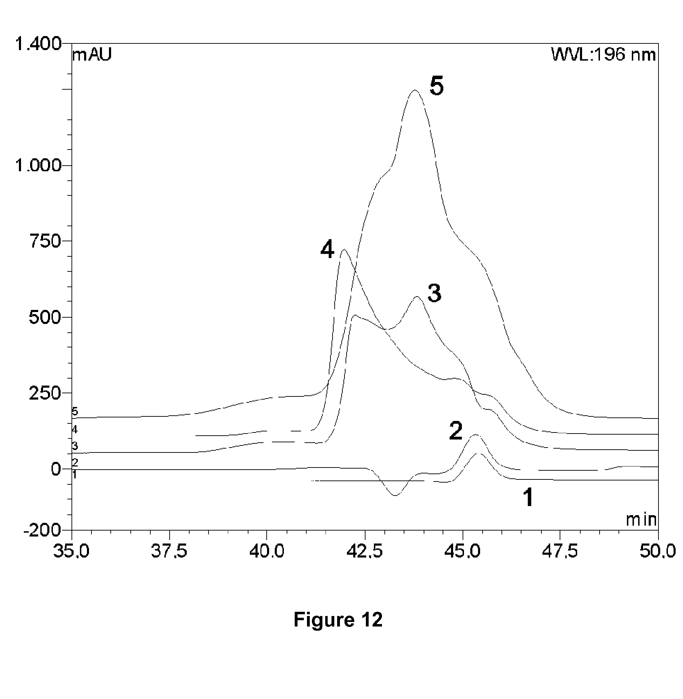

**Figure 12** shows the detection of complex formation between Gd³⁺ and polyP in aqueous solution. The polyP•Gd complexes can be separated from the free Gd³⁺ ions and polyP using a silica based Tosoh TSK G3000SW gel filtration column (Tosoh Bioscience LLC; 7.5 mm x 60 cm; 100 mM NaCl; 0.5 ml/min). Curve 1: polyP (sodium salt); Curve 2: 25 mM GdCl₃ plus 25 mM polyP; Curve 3: 10 mM GdCl₃ plus 25 mM polyP; Curve 4: 50 mM phosphate (potassium salt); Curve 5: 25 mM GdCl₃.

### Examples

In the experiments described below the inventors used concentrations for polyP and MeCl₃ which allow a higher discrimination/resolution of the data obtained and not to work at saturating or plateau levels of the compounds. Accordingly, the low concentrations of 5 µM polyP and of 5 µM polyP•Me were chosen. These concentrations are the threshold values of being ineffective and start to cause a biological effect. If not otherwise indicated, "Me" in MeCl₃ and polyP•Me of the experiments described herein was Gd (gadolinium).

### Effect of polyP•Me, MeCl₃ or polyP on proliferation/viability of SaOS-2 cells

The effects of polyP•Me as well as of the single components polyP and MeCl₃ on proliferation/viability of SaOS-2 cells were determined by applying the MMT colorimetric assay (MMT = 3-(4,5-dimethylthiazol-2-yl)-2,5-diphenyltetrazolium bromide). The data revealed that the viability/proliferation of SaOS-2 cells, deduced on the values of the extent of development of the dye formazan, did not change within the tested concentration range of 0.3 to 30 µM for MeCl₃, **polyP** or polyP•Me (Figure 2).

Figure 3 shows that addition of LaCl₃ in the absence or presence of polyP (50 µM) did not affect the cell number of SaOS-2 cells even at concentrations between 0.05 mM and 1 mM LaCl₃, while addition of AlCl₃ in the absence or presence of polyP (50 µM) resulted in a strong reduction of cell number at concentrations of 0.1 mM AlCl₃ and higher. Only at a very high concentration, LaCl₃ caused a strong drop of SaOS-2 cell number.

### Trivalent cation-induced mineralization in SaOS-2 cells: Alizarin Red staining

The influence of trivalent cations on the extent of mineralization of SaOS-2 cells was determined *in vitro* using McCoy's medium/10% FCS and applying Alizarin Red S as a dye to monitor HA formation. For the experiments shown here the concentrations of the test compounds (MeCl₃ or polyP) and polyP•Me were set to 5 µM. In the absence of a cocktail to activate mineralization the staining intensity was low (Figure 4A-a). Addition of the activation cocktail, consisting of ß-glycerophosphate/ascorbic acid/dexamethasone, to the cells increased the staining intensity significantly (Figure 4A-b). Addition of MeCl₃ or polyP (Ca²⁺ salt) intensified the staining intensity of the SaOS-2 cells in the presence of the activation cocktail (Figure 4A-c and Figure 4A-d). Even more, if the two agents were applied together, as polyP•Me, a strong increase of HA formation by the osteoblast-like cells could be measured (Figure 4A-e). The effect of enhanced HA formation was twice as high for polyP•Me, compared with polyP alone.

The same reagent, Alizarin Red S, was also applied to quantitatively monitor the effect of the compounds on HA in the liquid phase (Figure 4B). In the absence of an activation cocktail the amount of Alizarin Red S complex formed was lower than 0.03 nmoles/µg DNA (not shown in Figure 4B) and increased in the presence of the cocktail during the 5 days (d) incubation to 0.31±0.06 nmoles/µg (Figure 4B). The extent of mineralization substantially increased in activated SaOS-2 cells during an incubation period of 7 days if the test compounds were added simultaneously with the activation cocktail. The increase was already significant after a 3 days incubation period - and was even more pronounced after 7 days. Compared to polyP•Me (1.23±0.11 nmoles/µg, 7 days after the addition of the activation cocktail), the enhancement was less pronounced if the two components were added separately as MeCl₃ (0.61±0.08 nmoles/µg) or polyP (0.86±0.08 nmoles/µg).

Figure 5 shows the influence of LaCl₃, polyP, and polyP•La complex on HA mineralization in SaOS-2 cells. The results revealed addition of increasing concentrations of LaCl₃ in the absence of polyP resulted in a marked increase in HA formation at a concentration of 0.05 mM LaCl₃ (Figure 5A). At concentrations higher than 0.1 mM LaCl₃, the stimulatory effect started to decline, and was not observed any more at concentrations of 0.5 mM and higher. An additional stimulatory effect was observed after addition of polyP to the LaCl₃-containing assays, i.e. in the presence of equimolar concentrations of LaCl₃ (50 µM) and polyP (50 µM) (Figure 5B), in addition to the effect of polyP alone that caused a 2.5-fold increase in HA mineralization compared to the assay without polyP (Figure 5A). Similar results were obtained if the values were normalized by cell number (Figure 6).

### Fine structure of HA crystals on SaOS-2 cells

To assure that the increased Alizarin Red S staining is due to a higher HA (Ca-phosphate) deposition SEM analyses were performed. In the absence of the activation cocktail no nodules could be identified on the cells (Figure 7A). In contrast, if the cells were incubated with the cocktail for 5 days, distinct HA nodules could be identified (Figure 7B). In the presence of the activation cocktail the cultures were incubated with MeCl₃ (Figure 7C), polyP (Figure 7D), or both components together, as polyP•Me (Figure 7E). It is apparent that the density of the nodules in activated cultures incubated with MeCl₃ or with polyP alone was lower, compared to polyP•Me. At higher magnification it could be observed that the nodules consisted of irregularly arranged prism-like nanorods (Figures 7F to H). The average sizes of the crystallite nodules were larger in cultures treated with the two components together as polyP•Me (Figure 7H), than those which formed during exposure to the single components (Figures 7F and G). It often appears that the HA nodules tend to cluster together in the assay with polyP•Me, while - under the conditions used - the nodules on the cells treated with polyP or MeCl₃ remained distinctly isolated. This observation gives a hint that in the polyP•Me-treated cultures the cells formed a fiber reinforced network between them, perhaps based on collagen type I.

### Element analyses of the nodules formed

To clarify and ascertain that crystals seen by SEM were indeed at least mainly composed of Ca-phosphate EDX analyses were performed. The SaOS-2 cultures were incubated for 7 days in the presence of 5 µM polyP•Me (Figures 8 A and B). The EDX analysis of an area, including a part of a nodule, showed signal peaks for C, N, O and Na and also P and Ca, indicative for HA. A strong signal for Si originated from the Si wafer. In addition, the overall mapping of the surfaces of the nodule-forming SaOS-2 cells, incubated with polyP•Me, revealed that those cell regions were free from detectable aluminum or lanthanides. Line-scan analysis was performed through a HA nodule region (Figures 8 C and D) and revealed that the nodules were indeed composed of high levels of P and Ca, and also C. Again no Me signals could be recorded.

### Induction of ALP (alkaline phosphatase) activity

In the following experiments, the inventors could demonstrate that trivalent cations, complexed with polyP, are more potent inducers of ALP activity in comparison to MeCl₃ or polyP alone. Using the low concentration of 5 µM polyP•Me, it was measured that the stimulating effect of polyP•Me on ALP exceeded that of polyP or MeCl₃ by a factor of about two (Figure 9).

### Degradation of polyP by exposure to culture medium

To obtain a direct proof if the accessibility of the ALP is different to polyP•Me, compared to polyP, *in vitro* incubation studies with medium that had been collected from cultures of SaOS-2 cells, incubated for 5 days with 5 µM polyP•Me, were performed. Thereby, conditions had been used to obtain largest possible stimulation of ALP. The gel electrophoretic analyses revealed that polyP is only marginally, if at all, degraded by the medium during a 72 h incubation period (Figure 10). In contrast, the polymer polyP•Me is readily degraded after 72/96 h (Figure 10); after staining with toluidine blue it became visible that polyP samples did not degrade markedly, while complexed polyP•Me showed a high degradation into oligo-phosphates after 72 h or 96 h, respectively.

### Expression of BMP2 and collagen type I genes

Previous results of the inventors revealed that high concentrations (100 µM) of polyP cause a slight, but significant, increase of the steady-state expression of the *BMP2* (Wang XH, Schröder HC, Diehl-Seifert B, Kropf K, Schlosmacher U, Wiens M, Müller WEG (2012) Dual effect of inorganic polymeric phosphate/polyphosphate on osteoblasts and osteoclasts in vitro. J Tissue Engineer Regen Med; doi: 10.1002/term.1465) and also of *collagen type I.* According to the invention, described here, MeCl₃, either administered alone, or in complex with polyP is superior to polyP. Activated SaOS-2 cells were incubated with these compounds for 1 day up to 7 days, using the lower concentration of 5 µM for those compounds each (Figure 11 C). Applying the technique of qRT-PCR it was found that already at day 3 a significant upregulation (1.6-fold) of the steady-state level for *BMP2* could be measured in those cells which had been treated with polyP•Me. At day 5 in all assays shown here, a significant increase in the steady-state expression was seen, both for MeCl₃ alone (1.4-fold), polyP (1.4-fold) and also for polyP•Me (2.1-fold). While the expression level for *BMP2* remained unchanged in cultures, treated for 7 days with MeCl₃ and polyP, but further raised for polyP•Me (2.2-fold).

Since in SaOS-2 cells *BMP2* and *collagen type I* genes are expressed and induced, in parallel or sequentially, the qRT-PCR experiments were extended for *collagen type I* (Figure 11D). The qRT-PCR data show that 5 µM polyP•Me, in contrast to free Me³⁺ or polyP, considerably upregulates the expression of *collagen type I* at day 5 and day 7. In addition, Figure 11 D shows that there is a clear synergistic effect in the presence of both components, Me³⁺ and polyP, forming the polyP•Me complex. In order to strengthen the qRT-PCR results electron microscopic studies were performed to identify potentially formed collagen fibrils. The images show that SaOS-2 cells incubated with polyP did not show spindle-sized forms, while the cells treated with polyP•Me did not only form this morphological phenotype but also collagen fibrils (Figures 11A and B).

The complexes formed between Gd³⁺ and polyP can be separated from the individual components by chromatography using a Tosoh TSK G3000SW gel filtration chromatography column. As shown in Figure 12, the polyP•Gd complexes formed are clearly separated from the free Gd³⁺ ions and polyP.

As shown in Figure 1, the formation of the polyP•Me complex results either in an intramolecular twisting of the polyP chain or a cross-linkage of different polyP chains. Or even more complex and intriguing is a linkage formation between two, or - as possible for Me³⁺ - even three chains of polyP molecules (homophilic interaction) or one (two) polyP chain(s) and two (one) different polyanion(s) (heterophilic interaction). In Figure 1, the ALP-driven disintegration of polyP•Me is also shown. These structural considerations might help to explain the unexpected finding underling this invention that polyP•Me has a more potent biological function compared to, for example, the sodium salt of polyP, polyP•Na. It is easily conceivable that the trivalent Me³⁺ ions that are forming complexes with poly-, oligo- or mono-phosphate units can undergo more diversified linkages, compared to the corresponding Ca²⁺, Na⁺ or K⁺ salts.

According to this invention, polyP in the complex with Me³⁺ is delivered to bone cells, more specifically to osteoblasts, where the polymer is hydrolyzed into (at least) oligo-, or into mono-phosphate units and Me³⁺ (Figure 1). In turn, phosphate acts as a substrate for HA synthesis proceeding at the cell-surface, while Me³⁺ might interact, as described, with cell surface receptor(s), e.g. with the extracellular calcium-sensing receptor, a G-protein coupled receptor which senses extracellular levels of calcium ions. The extracellular calcium-sensing receptor is well known to exist on cell membranes of SaOS-2 cells, where it acts as a receiver for extracellular Ca²⁺ signals. Importantly, cells after stimulation with Me³⁺ via the extracellular calcium-sensing receptor induce gene expression of BMP2 and even a release of this mediator into the extracellular space (Figure 1).

### Application of polyP•Me in combination with polyP

A further aspect of this invention concerns the application of polyP•Me in combination with polyP, whereby the polyP can be present as a sodium salt or a salt with another alkali cation or as a complex with a divalent cation [polyP (Me²⁺ complex)], such as calcium [polyP (Ca²⁺ complex)]. The preparation of these complexes is state-of-the-art and has previously been described in EP 2 489 346.

The polyP•Me either alone or combined with polyP or its salts or complexes can be encapsulated in an organic polymer such as shellac, alginate, or poly(lactic acid), or poly(D,L-lactide)/polyvinyl pyrrolidone-based microspheres, following state-of-the art procedures, as previously described by the inventors in EP 2 489 346.

This invention also involves various formulations of polyP•Me either alone or combined with polyP or its salts or complexes.

### Application of polyP•Me in combination with monomeric or polymeric silicic acid

A further aspect of this invention concerns the combined application of polyP•Me and monomeric or polymeric silicic acid or one or more of the components (enzymes, proteins, and substrates) involved in their formation.

### Methods

### Cells and incubation conditions

Human osteogenic sarcoma SaOS-2 cells are cultured in McCoy's medium (Biochrom, Berlin; Germany) containing 5 mM Na-phosphate and 1 mM CaCl₂, supplemented with 15% heat inactivated fetal calf serum (FCS), Na-pyruvate (1 mM), Ca(NO₃)₂ (0.5 mM), penicillin (100 U/ml), and streptomycin (100 µg/ml), in 6-well plates (surface area 9.46 cm²; Orange Scientifique, Braine-I'Alleud; Belgium) in a humidified incubator at 37°C and 5% CO₂. Routinely, 2x10⁴ cells are added per well (total volume 3 ml).

Different concentrations of MeCl₃ are added from a stock solution of 200 µM; polyP is added as Ca²⁺ salt, complexed by addition of the Na-salt of polyP with Ca²⁺ at a stoichiometric molar ratio of 2 : 1 (based on phosphate). Separately, polyP•Me is prepared by mixing of the Na-salt of polyP with MeCl₃ in a 3:1 stoichiometric ratio.

### Cell proliferation/viability assays

SaOS-2 cells are seeded at a density of 2x10⁴ cells per 3-ml well in a 24-multi-well plate (Orange Scientifique) and cultured for 3 days in McCoy's medium/15% FCS. Increasing concentrations of MeCl₃, polyP or polyP•Me are added to the cultures. After incubation, cell proliferation is determined applying the colorimetric method based on the tetrazolium salt XTT.

### Induced mineralization in SaOS-2 cells

SaOS-2 cells are seeded in multi-well plates. After an incubation period of 2 days mineralization is induced with an activation cocktail, composed of 50 µM ascorbic acid, 10 nM dexamethasone, and 1 mM β-glycerophosphate. Immediately or after an incubation period of up to 7 days the extent of mineralization is assessed by staining with 10% Alizarin Red S (Schröder HC, Borejko A, Krasko A, Reiber A, Schwertner H, Müller WEG (2005) Mineralization of SaOS-2 cells on enzymatically (Silicatein) modified bioactive osteoblast-stimulating surfaces. J Biomed Mat Res Part B - Applied Biomaterials 75B:387-392). A quantitative determination of HA is likewise achieved with Alizarin Red S as a probing dye and by performing the reaction in solution (Müller WEG, Wang XH, Diehl-Seifert B, Kropf K, Schloßmacher U, Lieberwirth I, Glasser G, Wiens M and Schröder HC (2011) Inorganic polymeric phosphate/polyphosphate as an inducer of alkaline phosphatase and a modulator of intracellular Ca2+ level in osteoblasts (SaOS-2 cells) in vitro. Acta Biomaterialia 7:2661-2671). The moles of Alizarin Red S bound are determined after generating a calibration curve; the values are normalized to the total DNA amount that had been measured in parallel cultures, using the PicoGreen assay (Wiens M, Wang XH, Schloßmacher U, Lieberwirth I, Glasser G, Ushijima H, Schröder HC, Müller WEG (2010) Osteogenic potential of bio-silica on human osteoblast-like (SaOS-2) cells. Calcif Tissue Int 87:513-524).

### Digital light microscopy

The cell layers are photographed with a KEYENCE BZ-8000 epifluorescence microscope (KEYENCE, Neu-Isenburg; Germany) using a S-Plan-Fluor 20x lens.

### Scanning electron microscopy and energy-dispersive X-ray spectroscopy

Scanning electron microscopic (SEM) analysis is performed with a SU 8000 microscope (Hitachi High-Technologies Europe, Krefeld; Germany) and employed at low voltage (1 kV; suitable for analysis of inorganic morphological structures).

Details of the application of energy-dispersive X-ray spectroscopy (EDX) were given previously (Müller WEG, Wang XH, Diehl-Seifert B, Kropf K, Schloßmacher U, Lieberwirth I, Glasser G, Wiens M and Schröder HC (2011) Inorganic polymeric phosphate/polyphosphate as an inducer of alkaline phosphatase and a modulator of intracellular Ca2+ level in osteoblasts (SaOS-2 cells) in vitro. Acta Biomaterialia 7:2661-2671). The beam-deceleration mode is used to improve the scanning quality. The SEM is coupled to an XFlash 5010 detector, an X-ray detector allowing a simultaneous EDX-based elemental analysis. The mapping is performed by using the HyperMap technique, as described (Salge T, Terborg R (2009) EDS microanalysis with the silicon drift detector (CDD): innovative analysis options for mineralogical and material science application. Anadolu Univ J Sci Technol 10:45-55).

Prior to the analyses the samples are thoroughly washed with 50 mM Tris-HCl (pH 7.4; supplemented with 100 mM NaCl).

### Alkaline phosphatase assay

Alkaline phosphatase (ALP) is determined in extracts from SaOS-2 cells using a photometric assay (Majeska RJ, Rodan GA (1982) Alkaline phosphatase inhibition by para thyroid hormone and isoproterenol in a clonal rat osteosarcoma cell line: possible mediation by cAMP. Calcif Tissue Int 34:59-66). After incubation, the cells are washed with phosphate-buffered saline (PBS) and then homogenized in a 12 mM Tris/NaHCO₃ buffer (pH 6.8; with 1 vol.% of Triton X-100). After centrifugation (15,000g, 5 min, 4°C) the supernatant is collected for determination of protein and DNA concentration and of ALP activity, as described above. The enzyme assay (200 µl) is composed of 0.1 M 2-amino-2-methyl-1-propanol (pH 10.5), 2 mM MgCl₂ and the reagent 2 mM 4-nitrophenylphosphate; aliquots of 20 µl of cell extract each are added to the assays. After termination of the assay (10 min), the absorbance is measured at 410 nm. After establishment of a calibration curve (p-nitrophenol) the enzyme activity is quantified. Six parallel assays were performed and the mean values (±SD) are calculated.

### Degradation of polyP and incubation conditions

The medium is collected from SaOS-2 cells, incubated with 5 µM polyP•Me per 1 ml for 5 d at 37°C. Aliquots of this medium are added to 20 µg of polyP (Na⁺ salt) or 20 µg polyP•Me per 1 ml to 150 µl culture medium. The duration of the incubation is either 1 h (taken as a control), or 72/96 h; then the samples are subjected to gel electrophoresis. The size of the chain length of polyP is determined by gel electrophoresis using 7 M urea/16.5% polyacrylamide gels (Leyhausen G, Lorenz B, Zhu H, Geurtsen W, Bohnensack R, Müller WEG, Schröder HC (1998) Inorganic polyphosphate in human osteoblast-like cells. J Bone Mineral Res 13:803-812). The gels were stained with toluidine blue.

### Quantitative real-time RT-PCR analysis

Quantitative real-time PCR (qRT-PCR) determination of the expression of *BMP2* and *collagen type I* is performed. In brief, SaOS-2 cells are incubated as described; then the cells are harvested, total RNA is extracted and cleaned of possible DNA contamination by DNAse I treatment. After first-strand cDNA synthesis, using the M-MLV reverse transcriptase (RT) (Promega, Mannheim; Germany), approximately 5 µg of total RNA is used for qRT-PCR in a 40 µl reaction mixture in an iCycler (Bio-Rad, Hercules, CA). The reactions are run in triplicate using 1/10 serial dilutions. Then the samples are supplemented with the SYBR Green master mixture (ABgene, Hamburg; Germany) and 5 pmol of each primer pair for the following three transcripts: for the house keeping gene *GAPDH* (glyceraldehyde 3-phosphate dehydrogenase, GenBank accession number NM_002046.3) forward primer Fwd: 5'-ACTTTGTGAAGCTCATTTCCTGGTA-3' [nt₁₀₁₉ to nt₁₀₄₃] and reverse primer Rev: 5'-TTGCTGGGGCTGGTGGTCCA-3' (nt₁₁₁₇ to nt₁₁₃₆) (product size 118 bp); as well as for *BMP2* (NM_001200.2), Fwd: 5'-ACCCTTTGTACGTGGACTTC-3' (nt₁₆₈₁ to nt₁₇₀₀) and Rev: 5'-GTGGAGTTCAGATGATCAGC-3' (nt₁₇₈₅ to nt₁₈₀₄, 124 bp) and human *collagen type I* (NM_000088) Fwd: 5'-ATGCCTGGTGAACGTGGT-3' [nt₂₃₁₁ to nt₂₃₂₈] and Rev: 5'-AGGAGAGCCATCAGCACCT-3' [nt₂₃₉₇ to nt₂₃₇₉] (87 bp). The threshold position is set to 50.0 relative fluorescence units above PCR subtracted baseline for all runs. Expression levels are normalized to the reference gene *GAPDH.*

### Determination of the chain length of inorganic polyphosphate (polyP) molecules

The chain length of inorganic polyphosphate molecules was determined by gel electrophoresis using 7 M urea/16.5 % polyacrylamide gels as described in the literature (Leyhausen G, Lorenz B, Zhu H, Geurtsen W, Bohnensack R, Müller WEG, Schröder HC (1998) Inorganic polyphosphate in human osteoblast-like cells. J Bone Mineral Res 13:803-812).

### Further methods

The results are statistically evaluated using the paired Student's *t*-test.

The results depicted in Figures 2-11 were obtained with polyPs with an average chain length of approximately 40 phosphate units (obtained from Chemische Fabrik Budenheim, Budenheim; Germany), but similar results have been obtained for polyPs with an average chain length of 100 phosphate units and larger, as well as with an average chain length of 10 phosphate units and lower.

## Claims

1. A composition for use in medicine or as a dietary supplement, the composition comprising at least one complex or salt of trivalent metal cation (Me³⁺) with inorganic polyphosphate (polyP), wherein the trivalent metal cation (Me³⁺) is selected from the elements of the group consisting of Al, Ga, In, La, Ce, Pr, Nd, Sm, Eu, Gd, Tb, Dy, Ho, Er, Tm, Yb and Lu.

2. The composition of claim 1 for use in the prophylactic or therapeutic treatment of osteoporosis or bone disorder, and for bone and tissue synthesis and biomedical engineering.

3. The composition of any of claims 1 or 2, wherein the inorganic polyphosphate (polyP) molecules consist of an average number of from 2 to 1000 phosphate units, preferably from 4 to 100 phosphate units, more preferably from 10 to 70 phosphate units, and/or wherein the inorganic polyphosphate (polyP) molecules consist of linear polyphosphate chains.

4. The composition of any of the foregoing claims, wherein the stoichiometric ratio of the inorganic polyphosphate (polyP) and the trivalent metal cations (Me³⁺) in the complex or salt is from 5:1 to 1:1, preferably from 4:1 to 2:1, most preferably about 3:1.

5. The composition of any of the foregoing claims, wherein the trivalent metal cations (Me³⁺) are selected from the elements of the group consisting of Al, La, and Gd.

6. The composition of any of the foregoing claims further comprising calcium ions (Ca²⁺).

7. The composition of any of the foregoing claims further comprising silicic acid in the form of monomeric silicic acid, polymeric silicic acid or combinations thereof.

8. The composition of any of the foregoing claims further comprising pharmaceutically suitable carriers and/or additives selected from poly(lactic acid) (PLA) microspheres, poly(lactide-co-glycolide) (PLGA) microspheres, poly(lactide-co-glycolide-glucose) (PLG-GLU) microspheres, gelatin, calcium phosphate cements, poly(lactide-co-glycolide) (PLGA) nanoparticles, polyethylene oxide (PEO) microspheres, carbomer microspheres, chitosan microspheres, poly(lactide-co-caprolactone) microspheres, calcium phosphate microspheres, liposomes, alginate beads, chitosan beads, anionic copolymers based on methacrylic acic and methyl methacrylate, cationic copolymers based on dimethylaminoethyl methacrylate, butyl methacrylate and methyl methacrylate, copolymers of ethyl acrylate, methyl methacrylate and methacrylic acid ester with quaternary ammonium groups, or combinations of the afore-mentioned.

9. The composition of any of the foregoing claims formulated for oral, parenteral or topic administration or for administration by subcutaneous injection, intravenous injection, intraarterial injection, intraossal injection, intravertebral injection, intraarticular injection, intramuscular injection.

10. The composition of any of the foregoing claims containing the at least one complex or salt of trivalent metal cation (Me³⁺) with inorganic polyphosphate (polyP) in an amount of from 0.5 to 40 % by weight, preferably in an amount of from 1 to 30 % by weight, more preferably in an amount of from 2 to 20 % by weight, still more preferably in an amount of from 5 to 15 % by weight.

11. The use of a composition according to any of the foregoing claims for the prophylactic or therapeutic treatment of osteoporosis or bone disorder, and for bone and tissue synthesis and biomedical engineering.

12. A method of the prophylactic or therapeutic treatment of osteoporosis or bone disorder including the administration of the composition according to any of claims 1 to 10 to a human or animal.


SEQUENCE LISTING

<110> Chemische Fabrik Budenheim KG

<120> Composition for prophylaxis and treatment of bone disorders

<130> 130210EP-CHEMBU

<140> EP14164688.5
<141> 2014-04-15

<160> 6

<170> BiSSAP 1.3

<210> 1
<211> 25
<212> DNA
<213> Artificial Sequence

<220>
<223> GAPDH forward primer Fwd

<400> 1
actttgtgaa gctcatttcc tggta                                          25


<210> 2
<211> 20
<212> DNA
<213> Artificial Sequence

<220>
<223> GAPDH reverse primer Rev

<400> 2
ttgctggggc tggtggtcca                                                20


<210> 3
<211> 20
<212> DNA
<213> Artificial Sequence

<220>
<223> BMP2 Fwd

<400> 3
accctttgta cgtggacttc                                                20


<210> 4
<211> 20
<212> DNA
<213> Artificial Sequence

<220>
<223> BMP2 Rev

<400> 4
gtggagttca gatgatcagc                                                20

<210> 5
<211> 18
<212> DNA
<213> Artificial Sequence

<220>
<223> human collagen type I Fwd

<400> 5
atgcctggtg aacgtggt                                                    18


<210> 6
<211> 19
<212> DNA
<213> Artificial Sequence

<220>
<223> human collagen type I Rev

<400> 6
aggagagcca tcagcacct                                                   19